(19) Europäisches Patentamt
European Patent Office
Office européen des brevets

(11) **EP 2 763 654 B1**

(12) **EUROPEAN PATENT SPECIFICATION**

(45) Date of publication and mention
of the grant of the patent:
**29.04.2020  Bulletin 2020/18**

(21) Application number: **12759652.6**

(22) Date of filing: **11.09.2012**

(51) Int Cl.:
*A61K 9/00* (2006.01)   *A61K 31/5575* (2006.01)
*A61K 47/36* (2006.01)   *A61K 47/02* (2006.01)
*A61K 31/5377* (2006.01)   *A61K 31/728* (2006.01)
*A61P 27/06* (2006.01)

(86) International application number:
**PCT/EP2012/003810**

(87) International publication number:
**WO 2013/037479 (21.03.2013 Gazette 2013/12)**

(54) **OPHTHTALMIC COMPOSITIONS COMPRISING PROSTAGLANDIN F2 ALPHA DERIVATIVES AND HYALURONIC ACID**

OPHTHALMISCHE ZUSAMMENSETZUNGEN ENHALTEND PROSTAGLANDIN-F2-ALPHA-DERIVATE UND HYALURONSÄURE

COMPOSITIONS OPHTALMIQUES CONTENANT DES DÉRIVÉS DE PROSTAGLANDINE 2F ALPHA ET DE L'ACIDE HYALURONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2011  EP 11306137**

(43) Date of publication of application:
**13.08.2014  Bulletin 2014/33**

(73) Proprietor: **Unither Pharmaceuticals
80084 Amiens Cedex 2 (FR)**

(72) Inventor: **HADJ-SLIMANE, Réda
F-75015 Paris (FR)**

(74) Representative: **Ipside
6, Impasse Michel Labrousse
31100 Toulouse (FR)**

(56) References cited:
EP-A1- 1 321 144     EP-A1- 1 759 702
WO-A1-2009/125246   WO-A1-2010/111449
WO-A2-2009/013019   WO-A2-2010/130462
US-A1- 2010 104 654  US-A1- 2011 118 349

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF INVENTION**

The present invention relates to the treatment of ocular hypertension and glaucoma. More specifically, the present invention relates to a preservative-free composition for treating ocular hypertension and glaucoma, wherein said composition comprises at least one analogue of prostaglandin and at least one hyaluronic acid polymer. The invention also relates to a pharmaceutical composition and to a medicament comprising such a composition, and the use of same for treating ocular hypertension and/or glaucoma. The invention also relates to a process for manufacturing such a composition.

**BACKGROUND OF INVENTION**

Glaucoma is an eye disease characterized by damage to the optic nerve head and related visual field defects, often accompanied by elevated intraocular pressure (IOP). Glaucoma is characterized by the slow and progressive degeneration of retinal ganglion cells and optic nerve axons. The disease affects over 66 million people worldwide, causing bilateral blindness in 6.8 million.

One may divide glaucoma in primary glaucoma without known preceding cause, and secondary glaucoma. Primary glaucoma may be genetically determined. The term secondary glaucoma refers to glaucoma caused by some known antecedent or concomitant ocular diseases. Furthermore, primary glaucoma can be classified on anatomic basis into four major divisions: open-angle glaucoma, angle closure glaucoma, mixed glaucoma and congenital glaucoma. Primary open-angle is the most frequent type of glaucoma. Open-angle glaucoma represents a chronic, slowly progressive optic neuropathy, characterized by progressive excavation of the optic nerve head and a distinctive pattern of visual field defects. The disease is multifactorial in origin and is associated more closely with elevated intraocular pressure (IOP) resulting in the main from reduced drainage of aqueous humor.

There are several recognised risk factors for glaucoma, such as an increased intraocular pressure (IOP), aging, family history, high myopia, systemic hypertension, cardiovascular disease, migraine headaches, peripheral vasospasm and prior nerve damages. The visual damage incurred from glaucoma is considered irreversible. However, it can be treated if diagnosed at an early enough stage.

Medications involve inhibiting the inflow of aqueous humor, enhancing the outflow of aqueous humor, protecting the optic nerves and manipulating the osmotic pressure between plasma and the eyes.

Currently available medications for the treatment of glaucoma belong to several pharmacological classes, including β-adrenergic blockers (timolol maleate, carteolol, betaxolol), cholinergic agonists, carbonic anhydrase inhibitors (dorzolamide, brinzolamide) or adrenergic receptor blockers (brimonidine).

Another method of reducing IOP is by enhancing the outflow of humor from the eyes through the use of muscarinic acetylcholine receptor agonists. This mechanism is indirect, and involves a muscarinic acetylcholine receptor (M3)-mediated contraction of the ciliary muscle. The contraction causes the widening of the spaces in the trabecular meshwork. The newest class of drugs using this strategy is the prostaglandin F2α derivatives (such as, for example, unoprostone, latanoprost, travoprost, bimatoprost, tafluprost and the like). All these medications operate under a mechanism whereby the IOP is lowered. These therapies are typically administered as eye drops.

Prostaglandin analogues are chemical moieties, found in tissues or organs of humans, exhibiting a wide range of physiological activities. For example, latanoprost, also known as isopropyl-(Z)-7-[(1R,2R,3R,5S)-3,5-dihydroxy-2-[(3R)-3-hydroxy-5-phenylpentyl] cyclopentyl]-5-heptenoate, is a prostaglandin F2α receptor agonist, lowering the IOP by promoting an uveoscleral outflow of aqueous humor (Camras, C.B.; Podos, S.M.; Rosenthal, J.S.; Lee, P.Y.; Severin, C.H. "Multiple dosing of prostaglandin F2 alpha or epinephrine on cynomolgus monkey eyes. I. Aqueous humor dynamics" Invest. Ophthalmol. Vis. Sci. 1987, 28, 463). Such properties of latanoprost make it possible to show a lasting effect of controlling the IOP and a superior effect of lowering the IOP. In addition, the compliance in patients for this drug is high, and its safety has been established by its excellent ocular tolerance (Sudesh, S.; Cohen, E.J.; Rapuano, C.J.; Wilson, R.P. "Corneal toxicity associated with latanoprost" Arch. Ophthalmol. 1999, 117, 539).

Latanoprost, travoprost and bimatoprost have been introduced in the market under the trademarks of Xalatan®, Travatan®, and Lumigan® respectively, as ophthalmic eye drop solutions for the treatment of ocular hypertension and glaucoma. Xalatan® is to date, the most prescribed anti-glaucoma medicine in the world.

However, problems associated with prostaglandin analogues are their rather poor water solubility and their chemical instability especially in aqueous solutions. In order to solve these problems, ophthalmic formulations comprising prostaglandin analogues have been developed.

For example, most of the ophthalmic eye drops compositions comprising prostaglandin analogues use a preservative agent which, besides having antimicrobial properties on bacteria and fungi, also solubilizes the analogues of prostaglandin and stabilizes it at cold temperature. Preservative agents may act through an interaction with the prostaglandin analogue and/or through its homogeneous dispersion or dissolution into the eye drop solution.

Nowadays, the most commonly used preservative in commercially available ophthalmic solutions is benzalkonium chloride (BAC). BAC (alkyl dimethyl benzyl ammonium chloride) is a nitrogen-based quaternary ammonium compound demonstrating broad-spectrum antimicrobial activity. BAC is one of most used antimicrobial preservatives for ophthalmic formulations but it has been also widely used in the formation of ophthalmic microemulsions (see for example US 5,698,219) because of its positive charge which stabilizes the droplets. Xalatan® contains 0.005% latanoprost, and is packaged in multi-dose containers of 2.5 mL preserved by 0.02% BAC. Travatan® contains 0.004% travoprost, and is packaged in a multi-dose container of 2.5 or 5 mL preserved by 0.02% BAC. Lumigan® contains 0.01% or 0.03% bimatoprost, and is packaged in a multi-dose container of 3 mL preserved by 0.005% BAC.

Even though these medicines are very appreciated for the treatment of glaucoma, they have side effects, some of which may be due to the presence of the preservative agent. Alter few months of treatment of this chronic life-long disease, these medicine eye drops may start to irritate and wound the ocular surface.

Indeed, studies have shown the allergenic properties (Park, H.J.; Kang, H.A.; Lee, J.Y.; Kim, H.O. "Allergic contact dermatitis from benzalkonium chloride in antifungal year solution" Contact Derm. 2000, 42, 306) and the toxicity of BAC (Marple, B.; Roland, P.; Benninger, M. "Safety review of benzalkonium chloride as a preservative used in intranasal solutions: an overview of Conflicting data and opinions" Otolaryngol. Head Neck Surg. 2004, 130, 131). In addition, a study has shown that repeated use of BAC for a long term (at a concentration of 0.02%) distorts the cornea and induces irreversible eye damage (Swan, K.C. "Reactivity of the Ocular Tissues to Wetting Agents" Am. J. Ophthalmol. 1944, 27, 118). Finally, a more recent study conducted in 2009 showed that BAC induced resistance of *Pseudomonas aeruginosa* type bacteria to the antibiotic ciprofloxacin (Mc Cay, P.H.; Ocampo-Sosa A.A.; Fleming, G.T.A. "Effect of subinhibitory concentrations benzalkonium chloride of on the Competitiveness of Pseudomonas aeruginosa grown in continuous culture" Microbiology 2010, 156, 30).

All these side effects promote a medical trend to limit the use of preservatives agents in ophthalmic eye drops solutions by reducing their concentration or eliminating them from compositions.

EP 1 759 702 discloses an ophthalmic solution comprising latanoprost, hyaluronic acid and cyclodextrine.

WO 2010/111449 and US 2010/104654 both disclose intraocular solid drug delivery systems such as implants or microspheres containing hyaluronic acid.

US 2011/118349 discloses a composition comprising prostaglandin F2α and analogues for healing ocular lesions, which contains hyaluronic acid and is free from preservatives.

WO 2010/130462 discloses a phosphate-free pharmaceutical composition which comprises at least one prostaglandin analogue and which may contain hyaluronic acid. However, to the Applicant knowledge, no prostaglandin analogue composition which is free or essentially free of preservatives and wherein said prostaglandin analogue remains soluble, stable and bioavailable is currently on the market. Consequently, there is still a need in ophthalmologic prostaglandin analogue products which are at least as efficient as the commercial products, which present an enhanced chemical stability of prostaglandin analogues, which are less toxic, more physically and chemically stable than conventional products, i.e. which are stable overtime and which present a good tolerability for the patient.

More specifically, there is currently a need for a prostaglandin analogue composition, which remains stable at room temperature, thus avoiding its refrigeration. Surprisingly, the Applicant herein shows that the addition of hyaluronic acid to a prostaglandin composition leads to its stabilization at room temperature.

## SUMMARY

The present invention thus relates to a composition as defined in claim 1

This composition may be as defined in any of claims 2 to 7.

In one embodiment of the invention, the composition is stable overtime, which means that the composition meets the requirements of Recovery Test A and/or of Recovery Test B. In another embodiment of the invention, the composition remains stable after filtration, which means that the composition meets the requirements of Filtration Test C. In one embodiment of the invention, the hyaluronic acid has a molecular weight ranging from 1.6 to 3 MDa, more preferably from 1.6 to 2.4 MDa. In one embodiment, the composition comprises an amount of hyaluronic acid ranging from about 0.1 to about 0.15% w/v.

The at least one analogue of prostaglandin is selected from the group comprising 13,14-dihydro-17-phenyl-18,19,20-trinorprostaglandin F2αisopropylester (latanoprost), 20-ethyl prostaglandin F2α-(+)-fluprostenol isopropyl ester (travoprost), 13,14-dihydro-17-phenyl-18,19,20-trinorprostaglandin F2α-ethylamide (bimatoprost), or a mixture of two or more thereof. In one embodiment, the composition comprises an amount of at least one analogue of prostaglandin ranging from about 0.0001 to about 0.5%, in weight to the total volume of the composition, preferably from about 0.0005 to about 0.1% w/v, more preferably from about 0.001 to about 0.05% w/v.

In one embodiment of the invention, the composition further comprises another active ingredient, preferably another anti-glaucoma agent, more preferably timolol maleate.

In one embodiment of the invention, the composition comprises:

- Latanoprost in an amount ranging from about 0.001 to about 0.05% w/v, preferably from about 0.002 to about 0.01% w/v, more preferably of about 0.005% w/v;
- Hyaluronic acid, in an amount ranging from 0.1 to 0.2% w/v, more preferably of 0.1% w/v; said hyaluronic acid having a molecular weight ranging from 1.6 to 4 MDa, preferably from 1.6 to about 3 MDa, more preferably from 1.6 to about 2.4 MDa;
- Excipients, preferably sodium chloride, sodium dihydrogen phosphate dehydrate and disodium phosphate anhydrous; and
- Water.

Another object of the invention is a pharmaceutical composition comprising the composition as hereinabove described in association with at least one pharmaceutically acceptable excipient. Another object of the invention is a medicament comprising the composition of the invention.

Another object of the invention is a composition, a pharmaceutical composition or a medicament as hereinabove described for use in treating ocular hypertension and/or glaucoma in a subject in need thereof.

In one embodiment of the invention, the composition, pharmaceutical composition or medicament is for use in treating ocular hypertension and/or glaucoma in a subject in need thereof by topical administration to the eye, preferably being in the form of eye drops, eye ointment, ophthalmic gel, eyewash solution, mascara and the like. In another embodiment, the composition, pharmaceutical composition or medicament is administered at least once a day.

In one embodiment of the invention, the composition, pharmaceutical composition or medicament of the invention is packaged in a unit-dose or in a multi-dose form. In one embodiment, the unit-dose or in a multi-dose form is a unit-dose or in a multi-dose container made of LPDE.

Another object of the invention is a process for manufacturing the composition of the invention, comprising the steps of:

- mixing the hyaluronic acid or a salt thereof, the prostaglandin analogue and excipient;
- filtering the resulting composition; and
- packaging the filtered composition, preferably in a container moulded just before filling.

**DEFINITIONS**

In the present invention, the following terms have the following meanings:

- **"About"** preceding a figure means plus or less 10% of the value of said figure.

- **"Preservative-free composition":** refers to a composition free of preservative with antimicrobial properties, i.e. an agent having microbicidal or microstatic properties in the composition. As used herein, a microbicidal agent is an agent that kills microbes, whereas a microstatic agent only inhibits the growth of microbes. In one embodiment of the invention, the term "preservative-free" may also refer to a composition comprising a non-microbicidal and/or to a non-microstatic amount of an agent that is normally referred to as a preservative agent.
As far as this invention in concerned, preservatives with antimicrobial properties should be distinguished from aiding agents involved in maintaining the physicochemical characteristics of the active compound or the solution: examples of such aiding agents include, but are not limited to, EDTA and Vitamin E. Some aiding agents may be present in the composition of the invention. In an embodiment, the term "preservative-free" more specifically refers to a composition that does not comprise a preservative selected from the list comprising quaternary ammonium halides, wherein halide is preferably chloride or bromide, such as, for example, benzalkonium halide (such as, for example, BAC), benzododecinium halide, (such as, for example, benzododecinium bromide), benzethonium halide (such as, for example, benzethonium chloride), lauralkonium halide (such as, for example, lauralkonium chloride), cetrimide, hexadecyltrimethylammonium halide (such as, for example, hexadecyltrimethylammonium bromide), tetradecyltrimethylammonium halide (such as, for example, tetradecyltrimethylammonium bromide), dodecyltrimethylammonium halide (such as, for example, dodecyltrimethylammonium bromide), cetrimonium halide, behenalkonium halide, cetalkonium halide (such as, for example, cetalkonium chloride), cetethyldimonium halide, cetylpyridinium halide, benzododecinium halide, chlorallyl methenamine halide, myristalkonium halide, stearalkonium halide or a mixture of two or more thereof; boric acid and tartric acid.

- **"Ophthalmic composition"** refers to a composition intended to be administered to the eye.

- **"Prostaglandin analogue"**: refer to molecules that bind to a prostaglandin receptor.

- "Treating" refers to both therapeutic treatment and prophylactic or preventative measures; wherein the object is to prevent or slow down (lessen) the targeted pathologic condition or disorder. Those in need of treatment include those already with the disorder as well as those prone to have the disorder or those in whom the disorder is to be prevented. A subject is successfully "treated" for glaucoma or ocular hypertension if, after receiving an effective amount of a composition according to the present invention, the subject shows observable and/or measurable reduction in or absence of one or more of the following: reduction in one or more of the symptoms associated with glaucoma or ocular hypertension; and improvement in quality of life issues. The above parameters for assessing successful treatment and improvement in the disease are readily measurable by routine procedures familiar to a physician.

- **"Effective amount"** refers to the level or amount of agent that is aimed at, without causing significant negative or adverse side effects to the target, (1) delaying or preventing the onset of glaucoma and/or ocular hypertension; (2) slowing down or stopping the progression, aggravation, or deterioration of one or more symptoms of glaucoma and/or ocular hypertension; (3) bringing about ameliorations of the symptoms of glaucoma and/or ocular hypertension; (4) reducing the severity or incidence of glaucoma and/or ocular hypertension; or (5) curing glaucoma and/or ocular hypertension. An effective amount may be administered prior to the onset of glaucoma and/or ocular hypertension, for a prophylactic or preventive action. Alternatively or additionally, the effective amount may be administered alter initiation of glaucoma and/or ocular hypertension, for a therapeutic action.

- **"Sterile"** refers to a composition that is free or essentially free of any form of life, such as, for example free of living bacteria or other microorganisms.

- **"Pharmaceutically acceptable excipient"** refers to an excipient that does not produce an adverse, allergic or other untoward reaction when administered to an animal, preferably a human. It may include any and all solvents, dispersion media, coatings, antibacterial and antifungal agents, isotonic and absorption delaying agents and the like. For human administration, preparations should meet sterility, pyrogenicity, general safety and purity standards as required by FDA Office of Biologics standards.

- **"Stabilizing amount"** refers to the amount of hyaluronic acid present in a composition comprising a prostaglandin analogue that is aimed at stabilizing the prostaglandin analogue, i.e. the amount of hyaluronic acid leading to a desired recovery of the prostaglandin analogue after preservation of the composition for a specific time period at a specific temperature. More specifically, a stabilizing amount of hyaluronic acid refers to the amount leading to a composition which is stable overtime, i.e. a composition passing the Recovery Test A and/or Recovery Test B as hereunder described. Said amount may depend on the molecular weight of hyaluronic acid and/or on the amount of prostaglandin analogue in the composition.

- **"Subject"** refers to an animal, preferably a mammal, more preferably a human. In one embodiment of the invention, a subject may also refer to a pet, such as, for example, a dog, a cat, a guinea pig, a hamster, a rat, a mouse, a ferret, a rabbit and the like.

## DETAILED DESCRIPTION

The present invention thus relates to a composition as claimed in claim 1. The composition of the invention is an ophthalmic composition. In one embodiment, the composition is for treating, or for use in treating, ocular hypertension and/or glaucoma in a subject in need thereof.

The at least one hyaluronic acid or a salt thereof is acting in the composition as a stabilizing agent. Said hyaluronic acid or a salt thereof is present at a stabilizing amount.

Examples of hyaluronic acid salts that may be used in the present invention include, but are not limited to, sodium hyaluronate, zinc hyaluronate, calcium hyaluronate and potassium hyaluronate.

In one embodiment of the invention, the hyaluronic acid has a molecular weight ranging from about 1 600 000 to about 3 000 000 Da, more preferably from about 1 600 000 to about 2 400 000 Da.

Methods for determining the molecular weight of hyaluronic acid are well-known from the skilled artisan. In a preferred embodiment of the invention, the method used for determining the molecular weight of hyaluronic acid is the method described by Laurent (Laurent et al, Biochem. Biophys. Acta, 1960, 42, 476).

According to the method described by Laurent et al, hyaluronic acid molecular weight is calculated from limiting viscosity data, according to the following equation:

$$[\eta] = 0.0036 \, M^{0.78}$$

wherein $\eta$ is the limiting viscosity and M is the molecular weight of hyaluronic acid. Limiting viscosity $(\eta)$ is calculated at 25°C. The measurements are made over a concentration range of 0.15 to $5.6 \cdot 10^{-3}$ g/mL, using Ostwald viscosimeter with outflow times of 40 to 50 sec/mL and shear gradients of about 1000 $cm^{-1}$.

Hyaluronic acid is commercially available, such as, for example, from Shiseido (Japan) or Contipro (Czech Republic). In one embodiment of the invention, hyaluronic acid having a molecular weight ranging from 1 600 000 to 4 000 000 Da, preferably from 600 000 to about 3 000 000 Da, more preferably from 1 600 000 to about 2 400 000 Da is from Shisheido. In another embodiment of the invention, hyaluronic acid having a molecular weight ranging from 1 600 000 to 4 000 000 Da, preferably from 1 600 000 to about 3 000 000 Da, more preferably from 1 600 000 to about 2 400 000 Da is from Contipro.

In one embodiment of the invention, the composition comprises an amount of hyaluronic acid (FIA) ranging from about 0.1 to about 0.15% w/v.

In one embodiment of the invention, the stabilizing amount of hyaluronic acid (HA) ranges from 0.1 to about 0.15% w/v of a hyaluronic acid preferably having a molecular weight ranging from 1 600 000 to 4 000 000 Da, more preferably from 1 600 000 to about 3 000 000 Da, even more preferably from 1 600 000 to about 2 400 000 Da.

In one embodiment of the invention, the respective amounts of Hyaluronic acid and Prostaglandin range from about 1 : 0.005 to about 1 : 1, preferably from about 1 : 0.01 to about 1 : 0.5, more preferably from about 1 : 0.025 to about 1 : 0.25. Advantageously when the prostaglandin is latanoprost, the ratio hyaluronic acid : latanoprost in the composition ranges from about 1 : 0.005 to about 1 : 1, preferably from about 1 : 0.01 to about 1 : 0.5, more preferably from about 1 : 0.025 to about 1 : 0.25, even more preferably from about 1 : 0.025 to about 1 : 0.1, still even more preferably from about 1 : 0.03 to about 1 : 0.06. In one embodiment of the invention, the ratio hyaluronic acid : latanoprost in the composition is of about 1 : 0.033. In another embodiment of the invention, the ratio hyaluronic acid : latanoprost in the composition is of about 1 : 0.05.

In one embodiment of the invention, the ratio hyaluronic acid : travoprost in the composition ranges from about 1 : 0.005 to about 1 : 1, preferably from about 1 : 0.01 to about 1 : 0.5, more preferably from about 1 : 0.025 to about 1 : 0.25, even more preferably from about 1 : 0.025 to about 1 : 0.1, still even more preferably from about 1 : 0.025 to about 1 : 0.05. In one embodiment of the invention, the ratio hyaluronic acid : travoprost in the composition is of about 1 : 0.027.

In one embodiment of the invention, the ratio hyaluronic acid : bimatoprost in the composition ranges from about 1 : 0.005 to about 1 : 1, preferably from about 1 : 0.01 to about 1 : 0.5, more preferably from about 1 : 0.025 to about 1 : 0.25, even more preferably from about 1 : 0.05 to about 1 : 0.25, still even more preferably from about 1 : 0.1 to about 1 : 0.25. In one embodiment of the invention, the ratio hyaluronic acid : travoprost in the composition is of about 1 : 0.2.

The at least one prostaglandin analogue is a prostaglandin F2$\alpha$ analogue selected from the group comprising 13,14-dihydro-17-phenyl-18,19,20-trinorprostaglandin F2$\alpha$-isopropylester (latanoprost), 20-ethyl prostaglandin F2$\alpha$-(+)-fluprostenol isopropyl ester (travoprost), 17-phenyl-18,19,20-trinorprostaglandin F2$\alpha$-amide, 13,14-dihydro-17-phenyl-18,19,20-trinorprostaglandin F2$\alpha$-ethylamide (bimatoprost), or a mixture of two or more thereof.

Latanoprost, travoprost, bimatoprost, like most of the prostaglandin analogues, are almost insoluble in water.

In one embodiment of the invention, the composition comprises an amount, preferably an effective amount for treating or for use in treating ocular hypertension and/or glaucoma in a subject in need thereof, of a prostaglandin analogue ranging from about 0.001 and about 0.1% in weight to the total volume of the composition (w/v). In one embodiment of the invention, the amount, preferably the effective amount, of prostaglandin analogue in the composition ranges from about 0.0001 to about 0.5%, in weight to the total volume of the composition, preferably from about 0.0005 to about 0.1% w/v, more preferably from about 0.001 to about 0.05% w/v.

In one embodiment, the composition of the invention comprises latanoprost in an amount, preferably an effective amount ranging from about 0.0001 to about 0.5%, in weight to the total volume of the composition, preferably from about 0.0005 to about 0.1% w/v, more preferably from about 0.001 to about 0.05% w/v, even more preferably from about 0.002% to about 0.01% w/v and still even more preferably of about 0.005% w/v.

In one embodiment, the composition of the invention comprises travoprost in an amount, preferably an effective amount, ranging from about 0.0001 to about 0.5%, in weight to the total volume of the composition, preferably from about 0.0005 to about 0.1% w/v, more preferably from about 0.001 to about 0.05% w/v, even more preferably from about 0.002% to about 0.01% w/v and still even more preferably of about 0.004% w/v.

In one embodiment, the composition of the invention comprises bimatoprost in an amount, preferably an effective amount, ranging from about 0.0001 to about 0.5%, in weight to the total volume of the composition, preferably from about 0.0005 to about 0.1% w/v, more preferably from about 0.001 to about 0.05% w/v, even more preferably from about 0.005% to about 0.05% w/v, still even more preferably from about 0.01% to about 0.04% w/v and still even more preferably of about 0.03% w/v.

In one embodiment of the invention, the composition further comprises one or more additional active ingredient, preferably one or more additional anti-glaucoma agent.

Examples of additional active ingredient include, but are not limited to, β-blockers (such as, for example, timolol maleate or carteolol chloride), carbonic anhydrase inhibitors (such as, for example, dorzolamide chloride), and α-adrenergic agonists (such as, for example, brimonidine tartrate).

In one embodiment of the invention, the composition comprises at least one prostaglandin analogue with timolol maleate. In a first embodiment, the composition comprises latanoprost and timolol maleate. In a second embodiment, the composition comprises travoprost and timolol maleate. In a third embodiment, the composition comprises bimatoprost and timolol maleate.

In one embodiment of the invention, the amount of additional active ingredient, preferably of additional anti-glaucoma agent, ranges from about 0.1 to about 0.5% in weight to the total volume of the composition. In another embodiment of the invention, the amount of additional active ingredient ranges from about 0.1 to about 1% in weight to the total volume of the composition, preferably from about 0.4 to about 0.8% w/v, more preferably from about 0.5 to about 0.7% w/v.

In another embodiment of the invention, the composition comprises timolol, preferably timolol maleate, in an amount ranging from about 0.1 to about 1% in weight to the total volume of the composition, preferably from about 0.4 to about 0.8% w/v, more preferably from about 0.5 to about 0.7% w/v.

In one embodiment, the composition of the invention further comprises additional ingredients such as, for example, a decongestant, an eye muscle accommodator, an antiphlogistic/styptic, a vitamin, an amino acid, provided that the ingredient does not cause a problem such as eye irritation.

Examples of decongestant include, but are not limited to, epinephrine, epinephrine hydrochloride, ephedrine hydrochloride, tetrahydrozoline hydrochloride, naphazoline hydrochloride, naphazoline nitrate, phenylephrine hydrochloride, methyl ephedrine hydrochloride, and the like.

Examples of eye muscle accommodator include, but are not limited to, neostigmine methylsulfate and the like.

Examples of antiphlogistic/styptic include, but are not limited to, ε-aminocaproic acid, allantoin, berberine chloride, berberine sulfate, sodium azulene sulfonate, dipotassium glycyrrhizinate, zinc sulfate, zinc lactate, lysozyme chloride, and the like.

Examples of vitamin include, but are not limited to, flavin adenine dinucleotide sodium, cyanocobalamin, retinol acetate, retinol palmitate, pyridoxine hydrochloride, panthenol, calcium pantothenate, sodium pantothenate, tocopherol acetate, and the like.

Examples of amino acid include, but are not limited to, potassium L-asparaginate, magnesium L-asparaginate, magnesium/potassium L-asparaginate (such as, for example, an equal parts mixture), aminoethyl sulfonic acid, sodium chondroitin sulfate, and the like.

In one embodiment of the invention, the composition does not comprise carbomers and/or nanocapsule of hyaluronic acid.

In one embodiment of the invention, the composition comprises, or consists of:

- Latanoprost in an amount ranging from about 0.001 to about 0.05% w/v, preferably from about 0.002 to about 0.01% w/v, more preferably of about 0.005% w/v;
- Hyaluronic acid, in an amount ranging from 0.1 to 0.2% w/v, more preferably of 0.1% w/v; said hyaluronic acid having a molecular weight ranging from 1.6 to 4 million Dalton (MDa), preferably from 1.6 to about 3 MDa, more preferably from 1.6 to about 2.4 MDa; preferably, said composition comprises 0.1% w/v of 1.6 MDa hyaluronic acid;
- Excipients, preferably sodium chloride, sodium dihydrogen phosphate dehydrate and disodium phosphate anhydrous; and
- Water.

In one embodiment of the invention, the composition is stable overtime. By stable overtime is meant a composition characterized by the fact that the prostaglandin analogue recovery (in percent) during a specific period of time at a specific temperature is superior to a desired percentage.

In a first embodiment of the invention, a composition is stable overtime if it meets the requirements of the following Recovery Test A:

Recovery Test A:

Recovery Test A consists in measuring the prostaglandin analogue recovery in a prostaglandin analogue composition preserved in a glass container at 25°C during at least 3 months.

The prostaglandin concentration of the composition is measured at t=0, i.e. as soon as the composition has been prepared, the obtained value being named $C_0$.

Then, the glass container is preserved at a specific temperature for 3 months.

Then, at the end of this specific period of time (t=3 months), the prostaglandin analogue concentration of the composition is measured. The obtained value is named $C_3$.

The value $(C_3/C_0)$ x 100 is then calculated. It corresponds to the prostaglandin analogue recovery.

Measurement of the prostaglandin analogue concentration is performed by means of High Pressure Liquid Chromatography (HPLC) with pump LC9, such as, for example, an HPLC from Shimadzu equipped with an UV detector, using a mixture of two mobile phases and using Shimadzu class-VP software.

It is considered that a composition meets the Recovery Test A requirement, and is thus considered as stable overtime, if the prostaglandin analogue recovery is superior to about 80%, preferably to about 90%, and more preferably to about 95, 96, 97, 98, 99% or more.

In a second embodiment of the invention, a composition is stable overtime if it meets the requirements of the following Recovery Test B:

Recovery Test B:

Recovery Test B consists in measuring the prostaglandin analogue recovery in a prostaglandin analogue composition preserved in a glass container at 30-40°C during 3 months.

The prostaglandin concentration of the composition is measured at t=0, i.e. as soon as the composition has been prepared, the obtained value being named $C_0$.

Then, the glass container is preserved at a specific temperature for 3 months.

Then, at the end of this specific period of time (t=3 months), the prostaglandin analogue concentration of the composition is measured. The obtained value is named $C_3$.

The value $(C_3/C_0)$ x 100 is then calculated. It corresponds to the prostaglandin analogue recovery.

Measurement of the prostaglandin analogue concentration is performed by means of High Pressure Liquid Chromatography (HPLC) with pump LC9, such as, for example, an HPLC from Shimadzu equipped with an UV detector, using a mixture of two mobile phases and using Shimadzu class-VP software.

It is considered that a composition meets the Recovery Test B requirement, and is thus considered as stable overtime, if the prostaglandin analogue recovery is superior to 70 %, preferably to about 80, 86, 90%, and more preferably to 96, 96.5, 97, 97.5, 98% or more.

In one embodiment, the preservative-free ophthalmic composition of the invention may be stored at room temperature (room temperature herein refer to a temperature ranging from about 15 to about 30°C, preferably of about 25°C), including unit-dose containers, unit-dose pipettes and dispensers.

Stability studies have shown that a preservative-free ophthalmic latanoprost composition according to the invention is stable for a long time, such as, for example, at least three months, at least 6 months, at least one year, preferably two years and more preferably three years. The same result can be observed for compositions containing travoprost or bimatoprost (see Examples).

The present invention also relates to a pharmaceutical composition as claimed in claim 8. In one embodiment of the invention, said pharmaceutically acceptable excipient is water. In one embodiment, the pharmaceutical composition of the invention comprises the preservative-free composition as hereinabove described. In one embodiment of the invention, the pharmaceutical composition of the invention comprises an effective amount the at least one analogue of prostaglandin and/or a stabilizing amount of hyaluronic acid.

The present invention also relates to a medicament as claimed in claim 9. In one embodiment, the medicament of the invention comprises the preservative-free composition as hereinabove described. In one embodiment of the invention, the medicament of the invention comprises an effective amount the at least one analogue of prostaglandin and/or a stabilizing amount of hyaluronic acid.

The present invention also relates to a preservative-free ophthalmic composition for use in treating ocular hypertension and/or glaucoma in a subject in need thereof, wherein the ophthalmic composition is as claimed in any of claims 1 to 7.

Another object of the invention is thus a composition, a pharmaceutical composition or a medicament as hereinabove described for use in treating ocular hypertension and/or glaucoma in a subject in need thereof.

According to one embodiment of the invention, the composition, the pharmaceutical composition or the medicament is for ocular administration, preferably is topically administered to the eye of the subject.

Examples of formulations adapted for topical administration to the eye include, but are not limited to, eye drops, eye ointment, ophthalmic gel, eyewash solution and the like. In one embodiment of the invention, the composition, the pharmaceutical composition or the medicament of the invention is in the form of a mascara or a composition to be applied on the eyelashes.

In one embodiment of the invention, the composition, the pharmaceutical composition or the medicament of the invention is a solution, preferably an aqueous solution. In one embodiment, the pharmaceutical composition or the medicament of the invention does not comprise microspheres. In one embodiment of the invention, the composition, the pharmaceutical composition or the medicament is sterile.

In one embodiment of the invention, the ophthalmic composition further comprises conventional additives or excipients. Examples of additives or excipients that may be added to the composition of the invention include, but are not limited to buffering agents, tonicity agents, pH adjustors and antioxidant agents.

Examples of suitable buffering agents include, but are not limited to, sodium dihydrogen phosphate dihydrate, sodium dihydrogen phosphate monohydrate, sodium phosphate anhydrous, citric acid or a salt thereof (such as, for example, sodium citrate and the like), gluconic acid or a salt thereof (such as, for example, sodium gluconate and the like), acetic acid or a salt thereof (such as, for example, sodium acetate and the like), phosphoric or a salt thereof (such as, for example, sodium monohydrogen phosphate and the like), various amino acids such as glutamic acid and $\varepsilon$-aminocaproic acid and tris buffers, or any combination thereof. In one embodiment, the buffering agent is sodium dihydrogen phosphate monohydrate, disodium phosphate anhydrous or a mixture thereof. In another embodiment, the buffering agent is a mixture of sodium dihydrogen phosphate dehydrate and disodium hydrogen phosphate.

Specific examples of tonicity agents include, but are not limited to, glycerol, sorbitol, mannitol, propylene glycol, glycerin, erythriol, arabitol, xylitol, ribitol, galactitol, multitol, macrogol (such as, for example, macrogol 4000), lactitol and other sugar alcohols, sodium chloride, potassium chloride and calcium chloride. In a preferred embodiment, the tonicity agent is selected from the group comprising sorbitol (such as, for example, D-sorbitol), glycerol and macrogol 4000. In one embodiment of the invention, the tonicity agent is a hypertonicity agent, preferably is sodium chloride.

In one embodiment of the invention, the pH of the ophthalmic solution is adapted to prevent any irritation of the eye following administration, and ranges from about 4 to about 10, preferably from about 5.0 to about 8.0, and more preferably from about 6.2 to about 7.2. In one embodiment of the invention, a pH adjustor is used, such as, for example, phosphates or borates. Examples of pH adjustors that may be comprised in the composition of the invention include, but are not limited to, sodium hydroxide, potassium hydroxide, sodium carbonate, sodium hydrogen carbonate, sodium bicarbonate, hydrochloric acid, citric acid or a salt thereof (such as, for example, sodium citrate, sodium dihydrogen citrate and the like), phosphoric acid or a salt thereof (such as, for example, disodium hydrogen phosphate, potassium dihydrogen phosphate, and the like), acetic acid or a salt thereof (such as, for example, sodium acetate, ammonium acetate and the like), and tartaric acid and/or hydrochloric acid or a salt thereof. In a preferred embodiment, the pH adjustor is selected from the group comprising disodium hydrogen phosphate and sodium dihydrogen phosphate dehydrate.

In one embodiment of the invention, an antioxidant may be added to the composition in order to prevent prostaglandin oxidation. Specific examples of antioxidant agents include, but are not limited to, sodium nitrite, ascorbic acid, L-ascorbic acid stearate, sodium hydrogensulfite, $\alpha$-thioglycerin, erythorbic acid, cysteine hydrochloride, acetyl cysteine, ethylenediaminetetraacetic acid, citric acid, potassium dichloroisocyanurate, dibutylhydroxytoluene, 2,6-di-*t*butyl-4-methylphenol, soybean lecithin, sodium thioglycollate, sodium thiomalate, vitamin A, vitamin C, vitamin E, tocopherol, ascorbyl pasthyminate, sodium pyrosulfite, butylhydroxyanisole, 1,3-butyleneglycol, propyl gallate, 2-mercaptobenzimidazole and oxyquinoline sulfate. Preferably, the antioxidant agent is ethylenediaminetetraacetic acid (EDTA) or salts thereof.

In one embodiment of the invention, the composition comprises sodium dihydrogen phosphate monohydrate, sodium phosphate anhydrous and/or sodium chloride, preferably comprises sodium dihydrogen phosphate monohydrate, sodium phosphate anhydrous and sodium chloride.

In another embodiment, the composition of the invention comprises D-sorbitol, glycerol and/or macrogol. In one embodiment, the composition of the invention comprises D-sorbitol, Glycerol and/or macrogol in an amount ranging from about 1% to about 10% in weight to the total volume of the composition, preferably ranging from about 2% to about 5% (w/v), more preferably of about 4.5% (w/v).

In another embodiment of the invention, the composition comprises EDTA. In one embodiment, the composition comprises EDTA in an amount ranging from about 0.005% to about 0.05% in weight to the total weight of the composition, preferably from about 0.01% to about 0.1% (w/v), more preferably of about 0.05% (w/v).

In another embodiment of the invention, the composition of the invention comprises:

- sodium dihydrogen phosphate monohydrate, sodium phosphate anhydrous and sodium chloride; and
- at least one of D-sorbitol, glycerol and/or macrogol, preferably one of D-sorbitol, glycerol and/or macrogol; and
- optionally EDTA.

In one embodiment of the invention, the composition of the invention further comprises a vehicle. Preferably, said vehicle is water.

Described herein is also a method for treating ocular hypertension and/or glaucoma in a subject in need thereof, wherein said method comprises administering to the subject a preservative-free composition, pharmaceutical composition or medicament according to the invention.

The method comprises the topical administration to the eye of the subject of the preservative-free composition, pharmaceutical composition or medicament of the invention.

In one embodiment of the invention, the composition is administered to the subject at least once a week, preferably at least twice a week, preferably at least once a day.

In one embodiment of the invention, an amount of the composition ranging from about 0.05 to about 10 mL of the composition is administered to the subject, preferably from about 0.1 to about 2.5 mL, more preferably from about 0.15 to about 1 mL, and even more preferably from about 0.2 to about 0.5 mL.

The skilled artisan will appreciate that the amount of the composition to be administered as well as the administration route may depend on the severity of the symptoms of the glaucoma patient to be treated. A typical administration of the composition of the invention may be the topical administration of about one eye drop once per day.

In one embodiment of the invention, the subject is diagnosed with glaucoma and/or ocular hypertension.

In another embodiment, the subject is at risk of developing glaucoma and/or ocular hypertension. In a first embodiment, said risk corresponds to a familial predisposition to glaucoma and/or ocular hypertension, such as, for example, a genetic predisposition to glaucoma and/or ocular hypertension. In a second embodiment, said risk corresponds to the presence, in the subject, of an elevated IOP. Other examples of risk factors include, but are not limited to, aging, high myopia, systemic hypertension, cardiovascular disease, migraine headaches, peripheral vasoplasm and prior nerve damage.

In another embodiment of the invention, the subject presents an IOP ranging from about 21 mmHg to about 25 mmHg. In another embodiment of the invention, the subject presents an IOP ranging from about 25 mmHg to about 30 mmHg. In another embodiment of the invention, the subject presents an IOP superior to 30 mmHg.

The present invention also relates to a process for manufacturing the composition of the invention as claimed in claim 15.

In one embodiment, the process of the invention comprises mixing the prostaglandin analogue, hyaluronic acid or a salt thereof and excipients with a vehicle, preferably water, in a reactor.

In one embodiment of the invention, the mixing comprises three steps:

(a) mixing the excipients, preferably by gentle stirring, and optionally adjusting the pH of the resulting solution to a pH of about 4 to about 10, preferably to about 5 to about 8, more preferably to about 6.2 to about 7.2 using a pH adjustor;

(b) adding hyaluronic acid or a salt thereof to a portion of the solution obtained in step (a), preferably to 2 to 50% of the solution obtained in step (a), more preferably to about 5-20%, more preferably to about 10%; and mixing, preferably stirring the resulting solution; optionally, the resulting solution is refrigerated at about 4-8°C;

(c) adding the prostaglandin analogue to the solution obtained in step (b); and mixing, preferably stirring the resulting solution;

(d) adding to the solution of step (c) the remaining portion of the solution obtained in step (a); and mixing; and adjusting water to the completed final volume and stirring, such as, for example, for 75 minutes; and

(e) optionally adjusting the pH of the resulting solution to a pH of about 4 to about 10, preferably to about 5 to about 8, more preferably to about 6.2 to about 7.2 using a pH adjustor.

The resulting solution is then filtered.

In one embodiment of the invention, the composition is preferably filtered just before the administration to the subject, in order to avoid any microbial contamination of the subject.

In one embodiment of the invention, the composition of the invention is such that, after filtration, the filtration yield is of at least 75%, preferably of at least 80, 85, 90%, more preferably of at least 95, 96, 97, 98, 99% or more, wherein the yield corresponds to the percentage of recovered prostaglandin analogue, preferably, said filtration yield are measured after filtration with a 0.2 $\mu$M filter. Methods for determining the filtration yield are well-known from the skilled artisan. Examples of such a method include, but are not limited to, Test B as hereunder described.

In one embodiment of the invention, the composition of the invention remains stable after filtration, which means that the amount of prostaglandin is not substantially lowered after filtration of the composition of the invention. A prostaglandin composition is considered as remaining stable after filtration if it meets the requirement of the following Filtration Test C:

Filtration Test C

Filtration Test C consists in measuring the filtration recovery FR of the filtration of a composition of the invention through a 0.2 $\mu$M filter.

The prostaglandin concentration of the composition is measured at t=0, i.e. as soon as the composition has been prepared, the obtained value being named $C_0$.

Then, the composition is filtered through a 0.2 $\mu$M filter.

Then, after filtration, the prostaglandin analogue concentration of the composition is measured. The obtained value is named $C_F$.

The value $F_R$ is then calculated as follows: $(C_F/C_0)$ x 100.

Measurement of the prostaglandin analogue concentration is performed by means of High Pressure Liquid Chromatography (HPLC) with pump LC9, such as, for example, an HPLC from Shimadzu equipped with an UV detector, using a mixture of two mobile phases and using Shimadzu class-VP software.

It is considered that a composition remains stable after filtration if its FR is superior to 75%, preferably superior to 80, 85, 90%, more preferably superior to 95, 96, 97, 98, 99% or more.

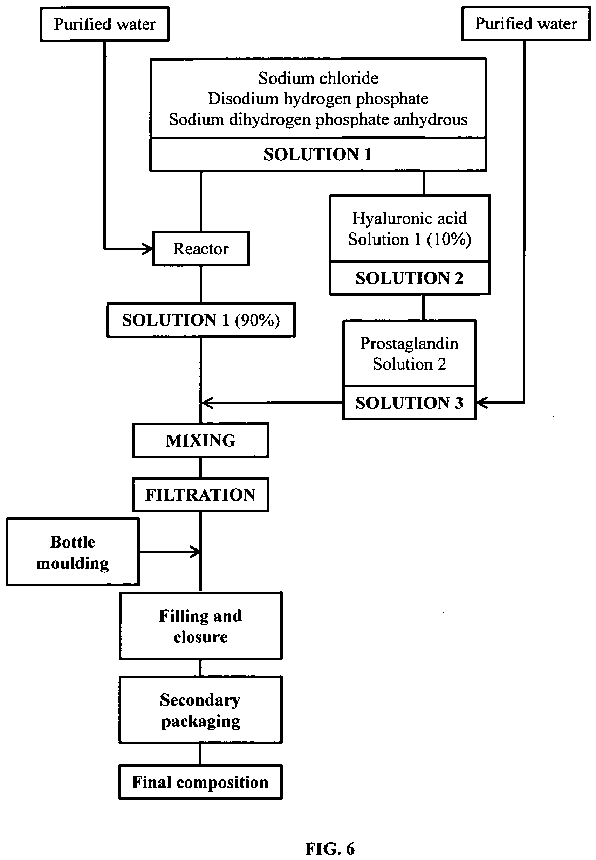

The composition of the invention is packaged after filtration. In one embodiment of the invention, the packaging, such as, for example, a container or a bottle manufactured for containing the composition of the invention, is moulded just before filling and closure. An example of a manufacturing process of the invention is shown in **Figure 6**.

In one embodiment of the invention, the composition is packaged in a unit-dose or in a multi-dose form, preferably in a unit-dose or in a multi-dose container. Preferably, the composition of the invention is packaged in a unit-dose container.

In one embodiment of the invention, the composition of the invention is packed in general plastic containers for eye drop preparations. Examples of materials that may be used for the container include, but are not limited to, a thermoplastic resin such as, for example polyethylene (PE), low density polyethylene (LDPE), polypropylene (PP), polyethylene terephthalate (PET), polyethylene naphthalate (PEN), polyallylate (PA); or a polycarbonate ethylene/vinyl alcohol copolymer. Preferably, the thermoplastic resin used is satisfactory in terms of cost, strength, optical transmittance, gas or water vapor barrier property (moisture permeation), and the like. In a preferred embodiment, the packaging is a unit-dose or multi-dose container produced in LDPE.

In one embodiment of the invention, the container comprises a filter on the top, preferably a 0.2 μM filter. Examples of material for the filter on the top of the container include, but are not limited to, polyether sulfone (PES), polyvinylidene fluoride (PVDF), polycarbonate, polytetrafluoroethylene (PTFE) and mixed cellulose ester (MSE). Preferably, the filter is made of polyether sulfone. Filters made from polyether sulfone are commercially available and include Millipore Express® and Millipore Express PLUS® made by Millipore Corporation.

In one embodiment of the invention, the ophthalmic compositions according to the invention may be conditioned in unit-dose containers or in multi-dose containers such as Comod® (Ursapharm Arzneimittel GmbH), Novelia® (Rexam), Ophthalmic Squeeze Dispenser (Aptar) or equivalent containers which allow supply of preservative-free ophthalmic solutions for long time. These airless multi-dose containers are fitted with a system preventing penetration of air in the bottle after instillation which could be responsible for microbial contamination of the sterile ophthalmic composition.

In one embodiment, the composition of the invention is packaged in a unit-dose container manufactured by blow moulding method.

In one embodiment, the unit-dose container has a volume ranging from about 0.1 to about 10 mL, preferably from about 0.5 to about 2.5 mL, more preferably of about 1 mL.

In one embodiment of the invention, the unit-dose container is filled with a volume of the composition of the invention ranging from about 0.05 to about 10 mL, preferably from 0.1 to about 1 mL, more preferably from about 0.2 to about 0.5 mL.

A large variety of shapes are known in such containers. Typical examples are seen in US 5,409,125 and US 6,241,124. In one embodiment of the invention, the container may have a cylindrical shape. It is possible to appropriately select a capacity of the container in the case of a multi-dose container.

In one embodiment of the invention, the multi-dose container comprising the composition of the invention has a capacity ranging from about 0.5 to about 20 mL, preferably from about 1 to about 10 mL, more preferably from about 2 to about 5 mL.

In one embodiment of the invention, the composition is packaged in unit-dose pipettes and dispensers.

The Applicant surprisingly showed that the presence of hyaluronic acid in an ophthalmic composition comprising a prostaglandin analogue leads to an improved stability of the prostaglandin analogue. Enhanced stability was measured in compositions preserved at room temperature or in stress conditions (temperature ranging of 30 or 40°C or more).

Consequently, the composition of the invention enhances the chemical stability of a prostaglandin analogue. Said prostaglandin analogue is selected from the group comprising latanoprost, bimatoprost and travoprost.

The composition of the present invention therefore presents the following advantages:

- it is stable at room temperature, for at least 1 month, preferably at least 2, 3, 4, 5 or 6 months, more preferably for at least 1, 2 or 3 years;
- it presents the same pharmacokinetic properties than Xalatan® when topically applied on the eye;
- it may easily be filtered;
- it does not comprise preservatives with antimicrobial properties, such as, for example, BAC, thus avoiding related toxicity;
- the prostaglandin analogue is efficiently solubilized in the composition.

## BRIEF DESCRIPTION OF THE DRAWINGS

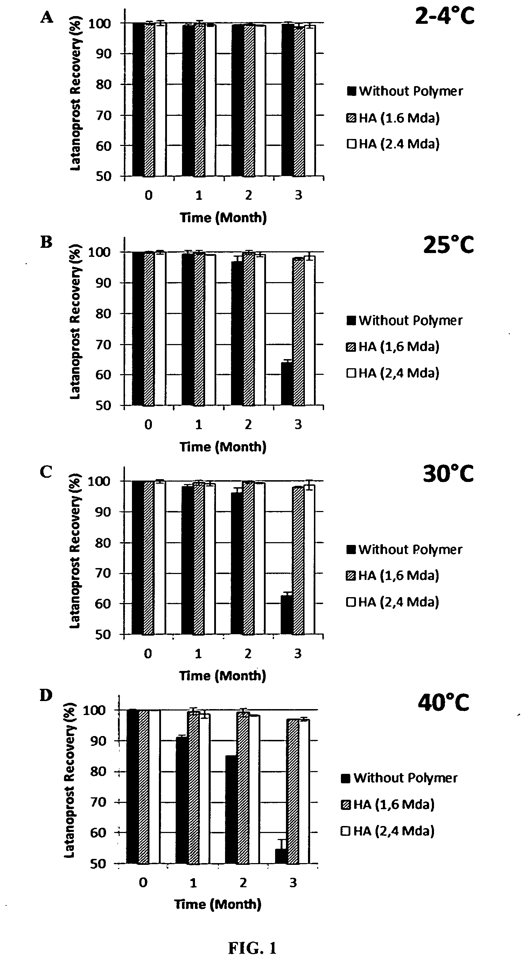

**Figure 1** is a combination of histograms showing the percentage of latanoprost recovery as a function of time, for latanoprost compositions comprising no hyaluronic acid (Without Polymer) or hyaluronic acid having a molecular weight of 1.6 MDa or 2.4 MDa. Latanoprost recovery was measured after preservation of the composition at 2-4°C (A), 25°C (B), 30°C (C) or 40°C (D).

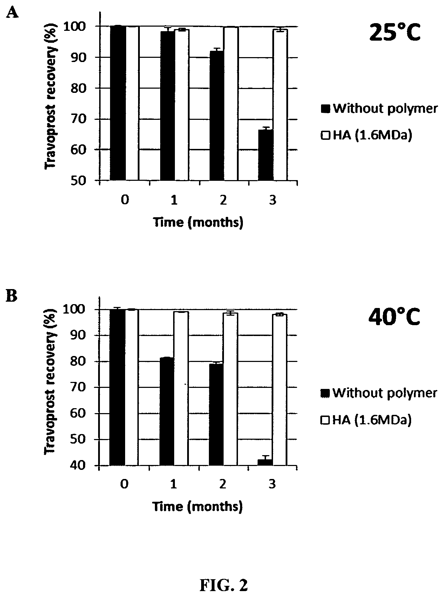

**Figure 2** is a combination of histograms showing the percentage of travoprost recovery as a function of time, for travoprost compositions comprising no hyaluronic acid (Without Polymer) or hyaluronic acid having a molecular weight of 1.6 MDa. Travoprost recovery was measured after preservation of the composition at 25°C (A) or 40°C (B).

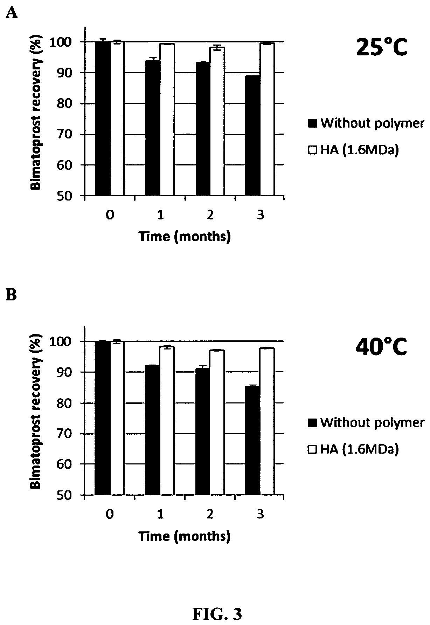

**Figure 3** is a combination of histograms showing the percentage of bimatoprost recovery as a function of time, for bimatoprost compositions comprising no hyaluronic acid (Without Polymer) or hyaluronic acid having a molecular weight of 1.6 MDa. Bimatoprost recovery was measured after preservation of the composition at 25°C (A) or 40°C (B).

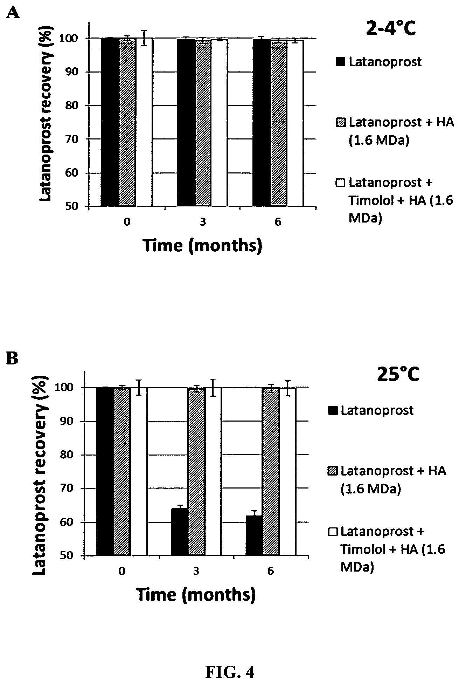

**Figure 4** is a combination of histograms showing the percentage of latanoprost recovery as a function of time, for compositions comprising latanoprost; latanoprost and 1.6MDa hyaluronic acid or latanoprost, timolol and 1.6 MDa hyaluronic acid. Latanoprost recovery was measured after preservation of the composition at 2-4°C (A) or 25°C (B).

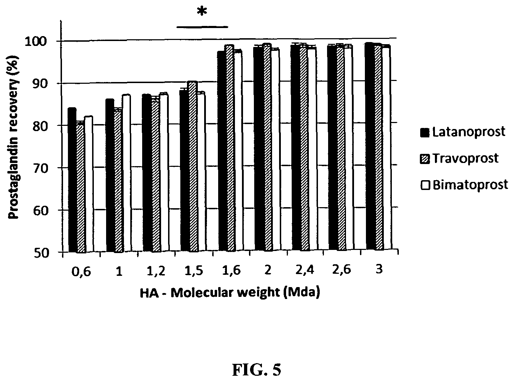

**Figure 5** is a histogram showing the percentage of prostaglandin recovery at 3 months as a function of the molecular weight of hyaluronic acid present in the prostaglandin composition. Tested prostaglandins are latanoprost, travoprost and bimatoprost. The percentage of 100% corresponds to the amount of prostaglandin present in the composition at t=0. (*): statistically significant difference (P<0.01).

**Figure 6** is a chart of a manufacturing process of the invention.

## EXAMPLES

The present invention is further illustrated by the following examples.

Example 1: Ophthalmic eye drop solutions

Different ophthalmic eye drop solutions are herein described as preferred compositions. However, such examples should by no means be interpreted in a limitative sense, but rather as possible realisation methods that may eventually be modified without thus being separated from the concept of this invention.

***Example of protocol used to prepare solutions containing a prostaglandin analogue:***

Disodium hydrogen phosphate anhydrous and sodium dihydrogen phosphate dihydrate were added to a solution of sodium chloride. After stirring 10 minutes, pH of the solution, called "solution 1", was measured and adjusted to 6.3-7.1 by using disodium phosphate solution at 10% or sodium dihydrogen phosphate solution at 10%. Then, hyaluronic acid was added to 10% of solution 1. The mixture obtained was stirred for 30 minutes or until complete dissolution to give a "solution 2", and refrigerated at least at 4-8°C. After that, the prostaglandin analogue was added. After stirring 30 minutes, the composition was mixed with the remaining 90% of solution 1 and purified water was used to complete final volume. Then, the composition was stirred for 75 minutes and pH was checked and corrected if necessary. Finally, sterile filtration was realised on membrane filter (0.2 $\mu$m) to obtain a clear and colourless solution free of particles.

A range of HA (0.6 to 3 MDa) was used to establish solubility and stability. All prostaglandin analogues samples were prepared using dilution of latanoprost (0.005 g), travoprost (0.004 g) or bimatoprost (0.03 g) in safe condition to obtain clear and colourless solution free of particles using phosphate buffer containing water, sodium dihydrogen phosphate monohydrate and disodium phosphate anhydrous as buffers, sodium chloride as hypertonicity agent, and in some compositions: D-Sorbitol or Macrogol 4000 as isotonic agent, and EDTA as antioxidant agent, with a control osmolarity (240-290 mosmol/kg) and equilibrated pH (6.3 to 7.1). Latanoprost, travoprost and bimatoprost were purchased from Finetech (Israel), Aceto (France) and Teva API Division (Italy). Hyaluronic acid was purchased from Shiseido (Japon) or Contipro (Czech Republic). Analyses were realized on ophthalmic compositions contained in unit-dose containers.

*Formula tested with 0.15% hyaluronic acid*

Table 1: Examples of compounds used in the ophthalmic eye drop solutions

| Substance | Function |
|---|---|
| Latanoprost | Active substance |
| Travoprost | Active substance |
| Bimatoprost | Active substance |
| Timolol (maleate) | Active substance |
| Hyaluronic Acid | Solubilizer and stabilizer |
| Sodium dihydrogen phosphate monohydrate | Buffering agent |
| Disodium phosphate anhydrous | Buffering agent |
| Sodium chloride | Hypertonicity agent |
| D-Sorbitol | Isotonic agent |
| Glycerol | Isotonic agent |
| Macrogol 4000 | Isotonic agent |
| Disodium edetate (EDTA) | Antioxidant agent |
| Purified water | Vehicle |

Table 2: Ophthalmic eye drop solutions comprising 0.005% Latanoprost with or without hyaluronic acid

| Substance | Composition A per 100 mL | Composition B per 100 mL | Composition C per 100 mL |
|---|---|---|---|
| Latanoprost | 0.005 g | 0.005 g | 0.005 g |
| Hyaluronic acid 1.6 MDa | - | 0.15 g | - |
| Hyaluronic acid 2.4 MDa | - | - | 0.15 g |
| Sodium dihydrogen phosphate monohydrate | 0.46 g | 0.46 g | 0.46 g |
| Disodium phosphate anhydrous | 0.474 g | 0.474 g | 0.474 g |
| Sodium chloride | 0.41 g | 0.41 g | 0.41 g |
| Purified water | Qs 100 mL | Qs 100 mL | Qs 100 mL |

Table 3: Ophthalmic eye drop solutions comprising 0.004% travoprost or 0.03% bimatoprost with 0.15% Hyaluronic Acid

| Substance | Composition D per 100 mL | Composition E per 100 mL |
|---|---|---|
| Travoprost | 0.004 g | - |
| Bimatoprost | - | 0.03 g |
| Hyaluronic acid 1.6 MDa | 0.15 g | 0.15 g |
| Sodium dihydrogen phosphate monohydrate | 0.46 g | 0.46 g |
| Disodium phosphate anhydrous | 0.474 g | 0.474 g |
| Sodium chloride | 0.41 g | 0.41 g |

(continued)

| Substance | Composition D per 100 mL | Composition E per 100 mL |
|---|---|---|
| Purified water | Qs 100 mL | Qs 100 mL |

Table 4: Ophthalmic eye drop solutions comprising 0.005% latanoprost, 0.004% travoprost, or 0.03% bimatoprost with 0.5% timolol maleate and 0.15% hyaluronic acid

| Substance | Composition F per 100 mL | Composition G per 100 mL | Composition H per 100 mL |
|---|---|---|---|
| Latanoprost | 0.005 g | - | - |
| Travoprost | - | 0.004 g | - |
| Bimatoprost | - | - | 0.03 g |
| Timolol maleate | 0.68 g | 0.68 g | 0.68 g |
| Hyaluronic acid 1.6 MDa | - | - | 0.15 g |
| Sodium dihydrogen phosphate monohydrate | 0.46 g | 0.46 g | 0.46 g |
| Disodium phosphate anhydrous | 0.474 g | 0.474 g | 0.474 g |
| Sodium chloride | 0.41 g | 0.41 g | 0.41 g |
| Purified water | Qs 100 mL | Qs 100 mL | Qs 100 mL |

**Formula tested with 0.10% hyaluronic acid**

Table 5: Ophthalmic eye drop solutions comprising 0.005% latanoprost with or without 0.5% timolol maleate, and 0.10% hyaluronic acid

| Substance | Composition I per 100 mL | Composition J per 100 mL |
|---|---|---|
| Latanoprost | 0.005 g | 0.005 g |
| Timolol maleate | - | 0.50 g |
| Hyaluronic acid 1.6 MDa | 0.10 g | 0.10 g |
| Sodium dihydrogen phosphate monohydrate | 0.52 g | 0.52 g |
| Disodium phosphate anhydrous | 0.474 g | 0.474 g |
| Sodium chloride | 0.41 g | 0.41 g |
| Purified water | Qs 100 mL | Qs 100 mL |

**Variation of excipients**

Table 6: Ophthalmic eye drop solutions comprising 0.005% latanoprost/0.10% hyaluronic acid with sorbitol, glycerol or Macrogol 4000

| Substance | Composition K per 100 mL | Composition L per 100 mL | Composition M per 100 mL |
|---|---|---|---|
| Latanoprost | 0.005 g | 0.005 g | 0.005 g |
| Hyaluronic acid 1.6 MDa | 0.100 g | 0.100 g | 0.100 g |
| Sodium dihydrogen phosphate monohydrate | 0.52 g | 0.52 g | 0.52 g |
| Disodium phosphate anhydrous | 0.474 g | 0.474 g | 0.474 g |
| Sodium chloride | 0.38 g | 0.38 g | 0.38 g |
| D-Sorbitol | 4.50 g | - | - |
| Glycerol | - | 4.50 g | - |
| Macrogol 4000 | - | - | 4.50 g |
| Purified water | Qs 100 mL | Qs 100 mL | Qs 100 mL |

Table 7: Ophthalmic eye drop solutions comprising 0.005% latanoprost/0.10% hyaluronic acid/EDTA with sorbitol, glycerol or Macrogol 4000

| Substance | Composition N per 100 mL | Composition O per 100 mL |
|---|---|---|
| Latanoprost | 0.005 g | 0.005 g |
| Hyaluronic acid 1.6 MDa | 0.100 g | 0.100 g |
| Sodium dihydrogen phosphate monohydrate | 0.52 g | 0.52 g |
| Disodium phosphate anhydrous | 0.474 g | 0.474 g |
| Sodium chloride | 0.38 g | 0.38 g |
| D-Sorbitol | 4.50 g | - |
| Macrogol 4000 | - | 4.50 g |
| Disodium edetate (EDTA) | 0.05 g | 0.05 g |
| Purified water | Qs 100 mL | Qs 100 mL |

Example 2: Chemical stability studies

In order to measure the intrinsic stability of the prostaglandin analogue in the compositions of the invention, the Applicant developed stability tests during which the compositions of the invention were subjected to specific temperatures (up to 40°C) during specified period of time. The stability study was performed on samples at different temperatures: in refrigeration 2-4°C (long-term stability test), at 25°C (accelerated stability test), at 30°C (stress stability test 1) and at 40°C (stress stability test 2).

Assessment of latanoprost, travoprost and bimatoprost recoveries was performed by means of High Pressure Liquid Chromatography (HPLC) with pump LC9. Briefly, samples were preserved in a glass container. Latanoprost, travoprost and bimatoprost contents were determined with an HPLC (Shimadzu) equipped with an UV detector and using a mixture of two mobile phases and using Shimadzu class-VP software.

Results are presented in Figures 1-4, wherein the percentage recovery of latanoprost, travoprost and/or bimatoprost are represented.

**Figure 1** represents the latanoprost recovery measured with Compositions A (without HA), B (HA 1.6 Mda) and C (HA 2.4 MDa). As shown in Figure 1A-D, the percentage recovery of latanoprost is increased in presence of hyaluronic acid, whatever the temperature. Moreover, this effect is observed for both tested molecular weight of hyaluronic acid, i.e. 1.6 MDa and 2.4 MDa.

As shown in **Figure 2**, this improvement of prostaglandin recovery by hyaluronic acid is also measured with travoprost (the composition with 1.6 MDa hyaluronic acid corresponds to composition D as hereinabove described).

Similar results were obtained for a composition comprising bimatoprost, as shown in **Figure 3**, wherein the composition with 1.6 MDa hyaluronic acid corresponds to composition E as hereinabove described.

Taken together, these results surprisingly showed that hyaluronic acid (either 1.6 MDa or 2.4 MDa) confers stability to a prostaglandin eye drop solution for at least 3 months and even under stress conditions (40°C).

Furthermore, a long term (6 months) recovery test at 2-4°C or 25°C was performed, using a composition comprising latanoprost (composition A); latanoprost and 1.6 MDa hyaluronic acid (composition I) or latanoprost, timolol and 1.6 MDa hyaluronic acid (composition J). Results are presented in **Figure 4**.

Surprisingly, as shown in **Figure 4**, the presence of hyaluronic acid (1.6 MDa) confers stability to the latanoprost and latanoprost/timolol eye drop solutions since after 6 months at 25°C.

Example 3: Chemical stability of prostaglandin in the presence of a range of molecular weight of hyaluronic acid

A range of hyaluronic acid molecular weight (0.6 to 3.0 million Dalton (MDa)) was used to study prostaglandin stabilities. Tested compositions comprised prostaglandin (0.005% latanoprost or 0.004% travoprost or 0.03% bimatoprost) and 0.15% (w/v) of hyaluronic acid in phosphate buffer. Assessment of latanoprost, travoprost and bimatoprost recoveries was performed by means of High Pressure Liquid Chromatography (HPLC). Briefly, samples were preserved in a glass container for 3 months at 40°C. Latanoprost, travoprost and bimatoprost contents were determined with an HPLC (Shimadzu) equipped with an UV detector and using a mixture of two mobile phases and using Shimadzu class-VP software.

Results are expressed as the percentage of prostaglandin recovery at 3 months (t=3 months) as compared to the initial concentration of prostaglandin (i.e. the percentage of prostaglandin recovery at t=0 is of 100%).

Results of the recovery test are presented in **Figure 5**. As shown in **Figure 5**, the percentage of prostaglandin recovery is statistically increased when the molecular weight of hyaluronic acid is superior or equal to 1.6 MDa. This effect is observed with the three tested prostaglandins, i.e. latanoprost, travoprost and bimatopropst.

Example 4: Comparative study of the ocular pharmacokinetics in pigmented rabbits

Comparative study of ocular pharmacokinetics has been realized in order to compare the persistence of latanoprost (acid form) in two ocular tissues (cornea and conjonctiva), after instillation of a single dose of three ophthalmic eye drop compositions containing latanoprost at 0.005%. The aim of the study is to evaluate whether composition A or composition B have an ocular kinetic profile equivalent to that of the commercial reference product Xalatan®.

Briefly, comparative study was realized on three groups of 9-pigmented rabbits. One drop of a 0.005% topical ophthalmic eye drop composition of latanoprost was instilled into the right eye of the rabbits (one drop = 50 µL), the left eye was used as negative control. The reference product containing preservative (Xalatan®) was administered to the first group and the other groups received the preservative-free ophthalmic eye drop solution (composition A for the group n° 2 and composition B for the group n° 3). Latanoprost (acid form) recovery was determined by HPLC after extraction from conjunctiva and cornea. Three rabbits per group were tested at each time of analysis.

Table 8: Ocular pharmacokinetic comparative study between Xalatan®, and composition A and composition B

| Formulation | Tissues | 0,5 h* | 6 h* | 24 h* |
|---|---|---|---|---|
| Xalatan® | Cornea | 100% | 5.8% | 0.3% |
| | Conjunctiva | 100% | 1.2% | 0.5% |
| Composition A | Cornea | 100% | 5.5% | 0.2% |
| | Conjunctiva | 100% | 1.1% | 0.4% |
| Composition B | Cornea | 100% | 5.7% | 0.3% |
| | Conjunctiva | 100% | 1.2% | 0.3% |
| * Samples were pooled from three animals per time point. | | | | |

These results show that the concentrations of remaining latanoprost in the cornea and in the conjunctiva both 6 and 24 hours post-instillation were equivalent between the three tested compositions, i.e. Xalatan®; composition A comprising latanoprost; and composition B comprising latanoprost and hyaluronic acid.

Therefore, the ocular concentration kinetics of latanoprost after single instillation of a drop of a 0.005% topical ophthalmic composition seem to be equivalent for the groups 1, 2 and 3.

It is known from the skilled artisan that topically administered latanoprost reaches the interior of the eye, such as, for example, the aqueous humor (Sjöquist et al, Drug Metab. Disp. 1998, 26, 745). Consequently, as the measured concentration kinetics are equivalent in the cornea and in the conjunctiva between Xalatan® and the composition of the invention (which all contain Latanoprost, and only differ by the presence of BAC or hyaluronic acid, respectively), the concentration kinetics in the interior in the eye, especially in the aqueous humor, are also equivalent.

Example 5: Clinical trial protocol

The principal objective of this clinical trial was to demonstrate the non-inferiority of the eye drops composition of the invention (comprising 50 µg/mL latanoprost and excipients without preservative) in comparison with the commercial latanoprost eye drops (Xalatan®, Pfizer) containing preservative, in the treatment of primary open angle glaucoma or intraocular hypertension by the average decrease of diurnal IOP measured between the first and last visit. Moreover, safety and tolerance of the composition of the invention were also assessed.

*Subjects*

Clinical trial was realized on 150 subjects (75 patients were randomized in each of the two treatment groups, receiving either the composition of the invention or Xalatan®). Subjects selected for this clinical trial were at least 18 years old, male or female, suffering from unilateral or bilateral primary open angle glaucoma or ocular hypertension. They were on treatment with an IOP-lowering prostaglandin analogue preparation for at least 6 months and possessed an IOP < 21 mmHg, with best-corrected visual acuity ≥ 20 of 100 corresponding to logMAR of 0.7 in both eyes.

*Methods*

Eye drops of either the composition of the invention or of Xalatan® were topically administered in the eye for 28 days. One drop of the composition was administered in the affected eyes once daily, in the evening (at 21 hours +/- 15 min).

Intraocular pressure was measured using, for example, the Goldmann applanation tonometer (which is the current gold standard instrument for measuring IOP). It measures IOP by flattening an area of the cornea and measuring the amount of pressure needed to flatten that area of the cornea. Because the instrument needs to come into contact with the eye, doctors first administered anaesthetic drops to numb the eye and fluorescein dye drops to make the tear film more visible during the measurement process.

Ophthalmic parameters (Visual acuity, intraocular pressure, ocular discomfort...) as well as general parameters (blood pressure, heart rate and the like) were assessed. Four visits were scheduled, from Day 1 (starting day) to Day 28 (Final visit). Evaluation of every participant in the clinical trial was performed by blinded investigators.

**Claims**

1. A composition intended for topical administration to the eye, comprising at least one analogue of prostaglandin selected from the group comprising latanoprost, bimatoprost and travoprost, as active compound, **characterized in that** said composition comprises a stabilizing amount ranging from 0.1 to 0.2% in weight to the total volume of the composition of at least one hyaluronic acid or a salt thereof having a molecular weight ranging from 1.6 to 4 MDa, said composition being free of preservatives with antimicrobial or microstatic properties, being cyclodextrine-free and being microspheres-free.

2. The composition according to claim 1, wherein the hyaluronic acid has a molecular weight ranging from 1.6 to 3 MDa.

3. The composition according to anyone of claims 1 to 2, comprising sodium dihydrogen phosphate monohydrate and/or disodium phosphate anhydrous.

4. The composition according to anyone of claims 1 to 3, comprising an amount of said at least one analogue of prostaglandin ranging from about 0.0001 to about 0.5%, in weight to the total volume of the composition.

5. The composition according to anyone of claims 1 to 4, further comprising another active ingredient.

6. The composition according to claim 5, further comprising timolol maleate.

7. The composition according to anyone of claims 1 to 6, comprising:

   - Latanoprost in an amount ranging from about 0.001 to about 0.05% w/v;
   - Excipients; and
   - Water.

8. A pharmaceutical composition comprising a composition according to anyone of claims 1 to 7 in association with at least one pharmaceutically acceptable excipient.

9. A medicament comprising a composition according to anyone of claims 1 to 7.

10. A composition according to anyone of claims 1 to 7, a pharmaceutical composition according to claim 8 or a medicament according to claim 9 for use in treating ocular hypertension and/or glaucoma in a subject in need thereof.

11. The composition, pharmaceutical composition or medicament according to claim 10, for use in treating ocular hypertension and/or glaucoma in a subject in need thereof by topical administration to the eye.

12. The composition, pharmaceutical composition or medicament according to anyone of claims 10 to 11, being administered at least once a day.

13. The composition, pharmaceutical composition or medicament according to anyone of claims 10 to 12, being packaged in a unit-dose or in a multi-dose form.

14. The composition, pharmaceutical composition or medicament according to claim 13, being packaged in a container made of low density polyethylene.

15. A process for manufacturing the composition according to anyone of claims 1 to 7, comprising the steps of:

   - mixing the hyaluronic acid or a salt thereof, the prostaglandin analogue and excipient;
   - filtering the resulting composition; and
   - packaging the filtered composition, preferably in a container moulded just before filling.

**Patentansprüche**

1. Zusammensetzung, die für eine topische Verabreichung in das Auge bestimmt ist, mindestens ein Analog von Prostaglandin umfassend, das aus der Gruppe ausgewählt ist, die Latanoprost, Bimatoprost und Travoprost als Wirkstoff enthält, **dadurch gekennzeichnet, dass** die Zusammensetzung eine Stabilisierungsmenge in einem Bereich von 0,1 bis 0,2 Gew.-% auf das Gesamtvolumen der Zusammensetzung von mindestens einer Hyaluronsäure oder eines Salzes davon umfasst, die ein Molekulargewicht in einem Bereich von 1,6 bis 4 MDa aufweist, wobei die Zusammensetzung frei von Konservierungsstoffen mit antimikrobiellen oder mikrostatischen Eigenschaften ist, frei von Cyclodextrin ist und frei von Mikrosphären ist.

2. Zusammensetzung nach Anspruch 1, wobei die Hyaluronsäure ein Molekulargewicht in einem Bereich von 1,6 bis 3 MDa aufweist.

3. Zusammensetzung nach einem der Ansprüche 1 bis 2, die Natriumdihydrogenphosphat-Monohydrat und/oder wasserfreies Dinatriumphosphat umfasst.

4. Zusammensetzung nach einem der Ansprüche 1 bis 3, umfassend eine Menge des mindestens einen Analogs von Prostaglandin in einem Bereich von etwa 0,0001 bis etwa 0,5 Gew.-% im Gesamtvolumen der Zusammensetzung.

5. Zusammensetzung nach einem der Ansprüche 1 bis 4, weiter einen anderen Wirkstoff umfassend.

6. Zusammensetzung nach Anspruch 5, weiter Timololmaleat umfassend.

7. Zusammensetzung nach einem der Ansprüche 1 bis 6, Folgendes umfassend:

- Latanoprost in einer Menge in einem Bereich von etwa 0,001 bis etwa 0,05% Gew./Vol.;
- Hilfsstoffe; und
- Wasser.

8. Pharmazeutische Zusammensetzung, umfassend eine Zusammensetzung nach einem der Ansprüche 1 bis 7 in Verbindung mit mindestens einem pharmazeutisch annehmbaren Hilfsstoff.

9. Medikament, umfassend eine Zusammensetzung nach einem der Ansprüche 1 bis 7.

10. Zusammensetzung nach einem der Ansprüche 1 bis 7, pharmazeutische Zusammensetzung nach Anspruch 8, oder Medikament nach Anspruch 9, zur Verwendung bei der Behandlung von Augenüberdruck und/oder Glaukoma bei einem davon betroffenen Patienten.

11. Zusammensetzung, pharmazeutische Zusammensetzung oder Medikament nach Anspruch 10, zur Verwendung bei der Behandlung von Augenüberdruck und/oder Glaukoma bei einem davon betroffenen Patienten durch topische Verabreichung in das Auge.

12. Zusammensetzung, pharmazeutische Zusammensetzung oder Medikament nach einem der Ansprüche 10 bis 11, die mindestens einmal am Tag verabreicht wird.

13. Zusammensetzung, pharmazeutische Zusammensetzung oder Medikament nach einem der Ansprüche 10 bis 12, die in einer Einheitsdosis- oder Mehr-Dosis-Form verpackt ist.

14. Zusammensetzung, pharmazeutische Zusammensetzung oder Medikament nach Anspruch 13, die in einem Behälter verpackt ist, der aus einem Low-Density Polyethylen gefertigt ist.

15. Verfahren zur Herstellung der Zusammensetzung nach einem der Ansprüche 1 bis 7, die folgenden Schritte umfassend:

- Mischen der Hyaluronsäure oder eines Salzes davon, des Prostaglandin-Analogs und einem Hilfsstoffes;
- Filtern der resultierenden Zusammensetzung; und
- Verpacken der gefilterten Zusammensetzung, vorzugsweise in einem Behälter, der unmittelbar vor dem Füllen geformt wird.

**Revendications**

1. Composition destinée à une administration topique à l'œil, comprenant au moins un analogue de prostaglandine choisi dans le groupe comprenant le latanoprost, le bimatoprost et le travoprost, en tant que composé actif, **caractérisée en ce que** ladite composition comprend une quantité stabilisatrice allant de 0,1 à 0,2 % en poids par rapport au volume total de la composition d'au moins un acide hyaluronique ou un de ses sels ayant un poids moléculaire allant de 1,6 à 4 MDa, ladite composition étant dépourvue de conservateurs à propriétés antimicrobiennes ou microstatiques, étant dépourvue de cyclodextrine et étant dépourvue de microsphères.

2. Composition selon la revendication 1, dans laquelle l'acide hyaluronique a une masse moléculaire allant de 1,6 à 3 MDa.

3. Composition selon l'une quelconque des revendications 1 à 2, comprenant du dihydrogénophosphate de sodium monohydraté et/ou du phosphate disodique anhydre.

4. Composition selon l'une quelconque des revendications 1 à 3, comprenant une quantité dudit au moins un analogue de prostaglandine allant d'environ 0,0001 à environ 0,5 %, en poids par rapport au volume total de la composition.

5. Composition selon l'une quelconque des revendications 1 à 4, comprenant en outre un autre ingrédient actif.

6. Composition selon la revendication 5, comprenant en outre du maléate de timolol.

7. Composition selon l'une quelconque des revendications 1 à 6, comprenant :

- du latanoprost dans une quantité allant d'environ 0,001 à environ 0,05 % p/v ;
- des excipients ; et
- de l'eau.

**8.** Composition pharmaceutique comprenant une composition selon l'une quelconque des revendications 1 à 7 en association avec au moins un excipient pharmaceutiquement acceptable.

**9.** Médicament comprenant une composition selon l'une quelconque des revendications 1 à 7.

**10.** Composition selon l'une quelconque des revendications 1 à 7, composition pharmaceutique selon la revendication 8 ou médicament selon la revendication 9, pour son utilisation dans le traitement de l'hypertension oculaire et/ou du glaucome chez un sujet en ayant besoin.

**11.** Composition, composition pharmaceutique ou médicament selon la revendication 10, pour son utilisation dans le traitement de l'hypertension oculaire et/ou du glaucome chez un sujet en ayant besoin par administration topique à l'œil.

**12.** Composition, composition pharmaceutique ou médicament selon l'une quelconque des revendications 10 à 11, qui est administré(e) au moins une fois par jour.

**13.** Composition, composition pharmaceutique ou médicament selon l'une quelconque des revendications 10 à 12, emballé (e) sous une forme unidose ou sous une forme multidose.

**14.** Composition, composition pharmaceutique ou médicament selon la revendication 13, emballé(e) dans un contenant formé en polyéthylène basse densité.

**15.** Procédé de fabrication de la composition selon l'une quelconque des revendications 1 à 7, comprenant les étapes de :

- mélange de l'acide hyaluronique ou d'un de ses sels, de l'analogue de prostaglandine et d'excipient ;
- filtration de la composition résultante ; et
- emballage de la composition filtrée, de préférence dans un contenant moulé juste avant remplissage.

**FIG. 1**

**A**

25°C

■ Without polymer

☐ HA (1.6MDa)

**B**

40°C

■ Without polymer

☐ HA (1.6MDa)

FIG. 2

**A**

25°C

■ Without polymer

□ HA (1.6MDa)

**B**

40°C

■ Without polymer

□ HA (1.6MDa)

FIG. 3

**A**

2-4°C

■ Latanoprost

▨ Latanoprost + HA (1.6 MDa)

☐ Latanoprost + Timolol + HA (1.6 MDa)

**B**

25°C

■ Latanoprost

▨ Latanoprost + HA (1.6 MDa)

☐ Latanoprost + Timolol + HA (1.6 MDa)

FIG. 4

FIG. 5

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5698219 A
- EP 1759702 A
- WO 2010111449 A
- US 2010104654 A
- US 2011118349 A
- WO 2010130462 A
- US 5409125 A
- US 6241124 B

**Non-patent literature cited in the description**

- **CAMRAS, C.B. ; PODOS, S.M. ; ROSENTHAL, J.S. ; LEE, P.Y. ; SEVERIN, C.H.** Multiple dosing of prostaglandin F2 alpha or epinephrine on cynomolgus monkey eyes. I. Aqueous humor dynamics. *Invest. Ophthalmol. Vis. Sci.,* 1987, vol. 28, 463
- **SUDESH, S. ; COHEN, E.J. ; RAPUANO, C.J. ; WILSON, R.P.** Corneal toxicity associated with latanoprost. *Arch. Ophthalmol.,* 1999, vol. 117, 539
- **PARK, H.J. ; KANG, H.A. ; LEE, J.Y. ; KIM, H.O.** Allergic contact dermatitis from benzalkonium chloride in antifungal year solution. *Contact Derm.,* 2000, vol. 42, 306
- **MARPLE, B. ; ROLAND, P. ; BENNINGER, M.** Safety review of benzalkonium chloride as a preservative used in intranasal solutions: an overview of Conflicting data and opinions. *Otolaryngol. Head Neck Surg.,* 2004, vol. 130, 131
- **SWAN, K.C.** Reactivity of the Ocular Tissues to Wetting Agents. *Am. J. Ophthalmol.,* 1944, vol. 27, 118
- **MC CAY, P.H. ; OCAMPO-SOSA A.A. ; FLEMING, G.T.A.** Effect of subinhibitory concentrations benzalkonium chloride of on the Competitiveness of Pseudomonas aeruginosa grown in continuous culture. *Microbiology,* 2010, vol. 156, 30
- **LAURENT et al.** *Biochem. Biophys. Acta,* 1960, vol. 42, 476
- **SJÖQUIST et al.** *Drug Metab. Disp.,* 1998, vol. 26, 745